# EUROPEAN PATENT APPLICATION

(11) **EP 3 970 634 A1**
(43) Date of publication of application: **23.03.2022**
(21) Application number: 21197170.0
(22) Date of filing: 16.09.2021
(51) Int. Cl.: A61B 17/12, A61B 18/12

(54) **LEFT ATRIAL APPENDAGE OCCLUDER DELIVERY DEVICE INCORPORATING ABLATION FUNCTIONALITY**

(30) Priority: 17.09.2020 US 202063079566 P
(71) Applicant: St. Jude Medical, Cardiology Division, Inc., St. Paul MN 55117 (US)
(72) Inventor: GABAY, Gregory, New Hope (US); TEGG, Troy, Elk River (US)
(74) Representative: Gill Jennings & Every LLP

(57) **Abstract**

A heart treatment device includes a delivery device allowing for simultaneous ablation of the left atrial appendage and delivery of an occluder into the left atrial appendage. The device includes a steerable catheter, an occluder releasably disposed within the catheter, an inflatable balloon coupled to a distal end of the catheter, and an array of electrodes coupled to the balloon. The balloon may be inflated to bring the electrodes into contact with the interior wall of the left atrial appendage. Energy supplied to the electrodes through the catheter ablates the tissue of the left atrial appendage to electrically isolate the left atrial appendage from the heart, and the delivery device deploys the occluder in the left atrial appendage.

## Description

### CROSS-REFERENCE TO RELATED APPLICATIONS

This application claims the benefit of the filing date of U.S. Provisional Application No. 63/079,566 filed September 17, 2020, the disclosure of which is hereby incorporated by reference herein.

### FIELD OF THE INVENTION

The present disclosure relates generally to the field of medical devices for treating certain vascular abnormalities. In particular, embodiments are directed to medical devices for occluding and ablating the left atrial appendage.

### BACKGROUND OF THE DISCLOSURE

The left atrial appendage (LAA) is a muscular pouch extending from the anterolateral wall of the left atrium of the heart. The LAA serves as a reservoir for the left atrium. During a normal cardiac cycle, the LAA contracts with the left atrium to pump blood from the LAA, which generally prevents blood from stagnating within the LAA. However, during cardiac cycles characterized by arrhythmias (e.g., atrial fibrillation), the LAA may fail to adequately contract. As a result, blood may stagnate within the LAA. Stagnant blood within the LAA is susceptible to coagulating and forming a thrombus, which can dislodge from the LAA and ultimately result in an embolic stroke.

Atrial fibrillation is an irregular and often rapid heart rate that commonly causes poor blood flow to the body. During atrial fibrillation, the heart's two upper chambers (the atria) beat chaotically and irregularly - out of coordination with the two lower chambers (the ventricles) of the heart.

A variety of devices and/or techniques, along with materials for and methods of manufacturing such devices, have been developed to occlude a vessel or an opening in an organ (e.g., heart) of a patient. Such devices, however, may not be particularly suited to address specific physiological conditions such as, e.g., occlusion of the LAA, in order to reduce the risk of embolisms when the patient is experiencing atrial fibrillation. During atrial fibrillation, the LAA may be a significant source of the undesirable formation of thrombiemboli. Also, occlusion of the LAA by surgical techniques may not always be possible and/or advisable. Further, some devices applied to occlude the LAA may be at risk of being expelled from the LAA due to the action of forces, such as those generated by atrial fibrillation. Moreover, some such devices may be configured such that they prevent subsequent alternate treatments of the condition such as, e.g., ablation therapy for atrial fibrillation.

Ablation procedures may be used to treat arrhythmias such as atrial tachycardia, atrial flutter, and atrial fibrillation. Energy is delivered from an ablation device to the endocardial and myocardial tissue. The energy delivered causes scarring of the tissue. The scars block impulses firing from within the tissue, thereby electrically "disconnecting" them or "isolating" them from the heart. In some cases, ablation procedures can provide restoration of normal heart rhythms.

Typically, occlusion and ablation therapy must be performed as separate operations or completed with the use of several devices. This complicates the treatment process and extends the time frame until treatment is completed. As such, there exists a need for an occlusion device and/or a device for deploying same that are capable of addressing the above-noted and other factors, and that simplify the processes of ablating the tissue of the LAA and deploying an occlusion device therein.

### BRIEF SUMMARY OF THE DISCLOSURE

Aspects of the present disclosure are directed to implantable medical devices, more particularly to implantable medical devices configured to occlude vessels, cavities, appendages, or the like, within a body, and deployment methods associated therewith.

This disclosure relates to devices and methods for the treatment of heart conditions. For example, this disclosure relates to devices and methods for simultaneously closing and ablating the left atrial appendage (LAA) to treat atrial fibrillation and to reduce the potential for embolic stroke. In addition, this disclosure relates to devices and methods for closing and ablating other body viscera, conduits, valves, and the like.

Devices and methods are described for occluding the LAA to exclude the LAA from blood flow to prevent blood from clotting within the LAA and subsequently embolizing, particularly in patients with atrial fibrillation. An LAA occlusion device is delivered via transcatheter delivery into the LAA and anchored by retention members on the surface of the body of the occlusion device. The device is collapsed for delivery and expands into place within the LAA to conform to the oval shape of the LAA with superior sealing effect. Thus, the device does not require an excessive number of sizes and negates the need for extensive pre-procedure imaging among other advantages.

Further described is a simultaneous ablation procedure using an inflatable balloon positioned at the distal end of a delivery catheter, the balloon having a mesh covering to couple an array of electrodes to the surface of the balloon. The balloon is configured to inflate via saline irrigation to contact the tissue of the LAA. Wires extend from an electronic source through the catheter to attach to the electrodes on the surface of the balloon. Ablation energy is delivered to electrically isolate the LAA.

According to a first aspect of the disclosure, a heart treatment device includes a catheter, an occluder, a balloon and an ablation electrode. The catheter may extend from a proximal end to a distal end. The occluder may be releasably disposed within the catheter and adapted to be deployed within a left atrial appendage of a patient. The balloon may be coupled to the catheter. The ablation electrode may be disposed on the balloon. The ablation electrode may be configured to deliver ablation energy to tissue of the left atrial appendage.

According to another embodiment of the disclosure, a heart treatment device includes a catheter, an occluder, an ablation electrode and a conductive wire. The catheter may extend from a proximal end to a distal end. The occluder may be disposed within the catheter and adapted to be deployed from the distal end of the catheter within a left atrial appendage of a patient. The ablation electrode may be disposed on the occluder and configured to deliver ablation energy to tissue of the left atrial appendage. The conductive wire may connect the electrode to an energy source.

According to another embodiment of the disclosure, a method for ablating tissue of the left atrial appendage of a patient and delivering a heart treatment device to the left atrial appendage includes navigating a catheter through a patient's body to approach the left atrial appendage. The catheter may have a distal end, a balloon coupled to the distal end, an ablation electrode disposed on the balloon and an occluder disposed within a lumen of the catheter. The balloon may be inflated until the electrode contacts tissue of the left atrial appendage. The tissue of the left atrial appendage may be ablated using ablation energy delivered to the ablation electrode. The occluder may be deployed from the lumen of the catheter into the left atrial appendage.

According to another embodiment of the disclosure, a method for ablating tissue of the left atrial appendage of a patient and delivering a heart treatment device to the left atrial appendage includes navigating a catheter through a patient's body to approach the left atrial appendage. An occluder disposed within a lumen of the catheter may be deployed into the left atrial appendage. Ablation energy may be delivered to the left atrial appendage through a wire connecting an energy source to an electrode in the left atrial appendage.

### BRIEF DESCRIPTION OF THE DRAWINGS

Fig. 1 is a schematic cutaway view of a human heart showing transapical and transseptal delivery approaches.
Fig. 2 is a schematic representation of the mitral valve, left atrial appendage and associated structures during normal operation.
Fig. 3 is a schematic side view of a left atrial appendage occluder having an array of electrodes disposed around its surface.
Fig. 4 is a schematic side view of a delivery device for the occluder of Fig. 3.
Fig. 5 is a schematic side view of the delivery device of Fig. 4 prepared for delivery with the occluder of Fig. 3 disposed therein.
Fig. 6 is a schematic side view showing the delivery device of Fig. 4 deploying the occluder of Fig. 3.
Fig. 7 is a schematic view showing the delivery device of Fig. 4 in the heart after transseptally delivering the occluder of Fig. 3 into the left atrial appendage.
Fig. 8 is a schematic side view of the distal end of a delivery device incorporating a balloon in a deflated state.
Fig. 9 is a schematic side view showing the delivery device of Fig. 8 with the balloon in an inflated state.
Fig. 10 is a schematic representation of the delivery device of Fig. 9 and an occluder within the left atrial appendage.
Fig. 11 is a schematic view of a flattened woven mesh configured to overlay the balloon shown in Fig. 9.
Figs. 12A-C are cross-sectional views showing embodiments of electrodes with different structures for coupling to the mesh of Fig. 11.
Fig. 13 is a schematic side view showing the delivery device of Fig. 9 with the balloon covered by the mesh of Fig. 11.
Fig. 14 is a schematic representation of an occluder within the left atrial appendage and an occluder delivery device within the left atrium and configured to ablate tissue via irreversible electroporation.

### DETAILED DESCRIPTION

The present disclosure will now be made with reference to the accompanying drawings in which some, but not all aspects of the disclosure are shown. Indeed, aspects of the disclosure may be embodied in many different forms and should not be construed as being limited to the aspects set forth herein. Like numbers refer to like elements throughout.

Aspects of the present disclosure provide a medical device for use in treating a target site within the body, such as occluding various vascular abnormalities, including, for example, the left atrial appendage (LAA). It is understood that the use of the term "target site" is not meant to be limiting, as the device may be configured to treat any target site, such as an abnormality, a vessel, an organ, an opening, a chamber, a channel, a hole, a cavity, or the like, located anywhere in the body. For example, the abnormality could be any abnormality that affects the shape or the function of a native lumen, such as an aneurysm, a congenital defect, a vessel dissection, flow abnormality or a tumor. Furthermore, the term "lumen" is also not meant to be limiting, as the abnormality may reside in a variety of locations within the vasculature, such as a vessel, an artery, a vein, a passageway, an organ, a cavity, a septum, or the like.

As used herein, the term "proximal," when used in connection with a delivery device or components of a delivery device, refers to the end of the device closer to the user of the device when the device is being used as intended. On the other hand, the term "distal," when used in connection with a delivery device or components of a delivery device, refers to the end of the device farther away from the user when the device is being used as intended. As used herein, the terms "substantially," "generally," "approximately," and "about" are intended to mean that slight deviations from absolute are included within the scope of the term so modified.

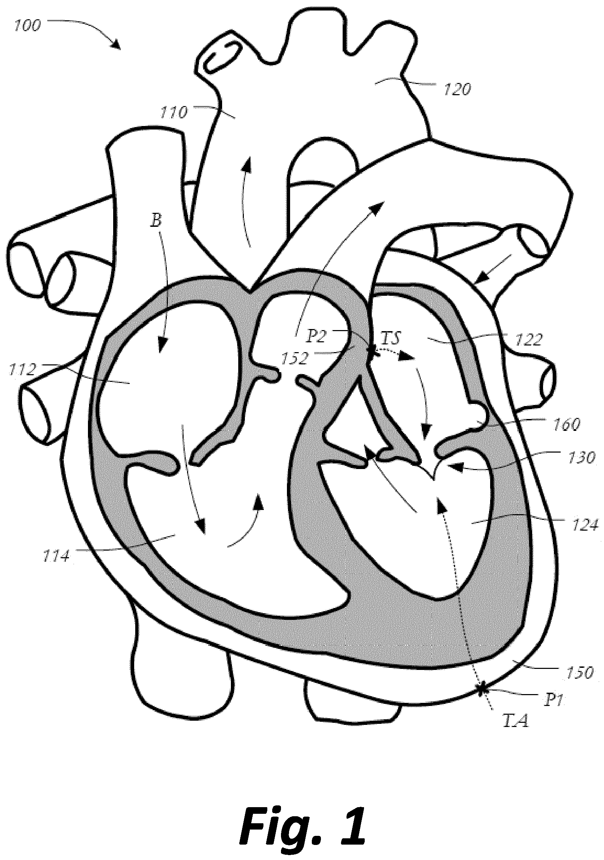

Fig. 1 is a schematic cutaway view of a human heart 100. The human heart includes two atria and two ventricles: a right atrium 112 and a left atrium 122, and a right ventricle 114 and a left ventricle 124. Heart 100 further includes an aorta 110, and an aortic arch 120. Disposed between left atrium 122 and left ventricle 124 is the mitral valve 130. Mitral valve 130, also known as the bicuspid valve or left atrioventricular valve, is a dual-flap that opens as a result of increased pressure from left atrium 122 as it fills with blood. As pressure in left atrium 122 increases above that in left ventricle 124, mitral valve 130 opens and blood passes toward left ventricle 124. Blood flows through heart 100 in the direction shown by arrows "B". Adjacent mitral valve 130 is LAA 160, which empties into left atrium 122.

A dashed arrow, labeled TA, indicates a transapical approach for treating or replacing heart tissue. In a transapical delivery of an occluder to LAA 160, a small incision is made between the ribs and into the apex of left ventricle 124 at position P1 in heart wall 150 to deliver the occluder to the target site. An alternative path, shown with a second dashed arrow labeled TS, indicates a transseptal approach with an incision made through the interatrial septum 152 of heart 100 from right atrium 112 to left atrium 122 at position P2. In a transseptal approach, the delivery system may enter the patient through a jugular vein (not shown), proceed through the superior vena cava (not shown) and into right atrium 112, pierce interatrial septum 152 into left atrium 122 and approach LAA 160.

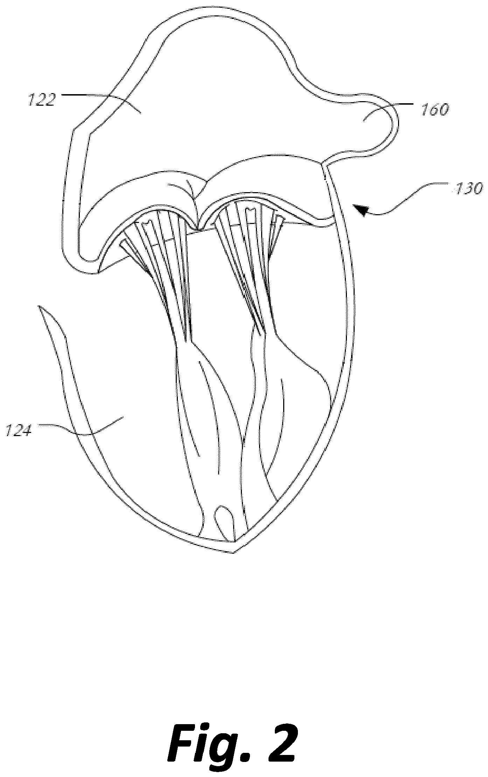

Fig. 2 is a more detailed schematic representation of left atrium 122 and left ventricle 124, more closely illustrating LAA 160. During normal function, LAA 160 contracts rhythmically along with left atrium 122 and blood from the LAA is ejected into the left atrium, and then passes through mitral valve 130 into left ventricle 124. With each cycle, blood in LAA 160 is completely emptied out and mitral valve 130 prevents backflow from left ventricle 124 to left atrium 122.

In some patients (e.g., older patients), right atrium 112 and left atrium 122 of heart 100 may not beat regularly, a condition known as atrial fibrillation. In some instances, this may result in partial or incomplete ejection of blood from LAA 160. Stagnant blood in LAA 160 may form clots, which may ultimately travel to the brain and cause a stroke. To prevent stagnant blood from remaining in and clotting in LAA 160, an occlusion device discussed in more detail below can be inserted as a plug in the cavity of the LAA.

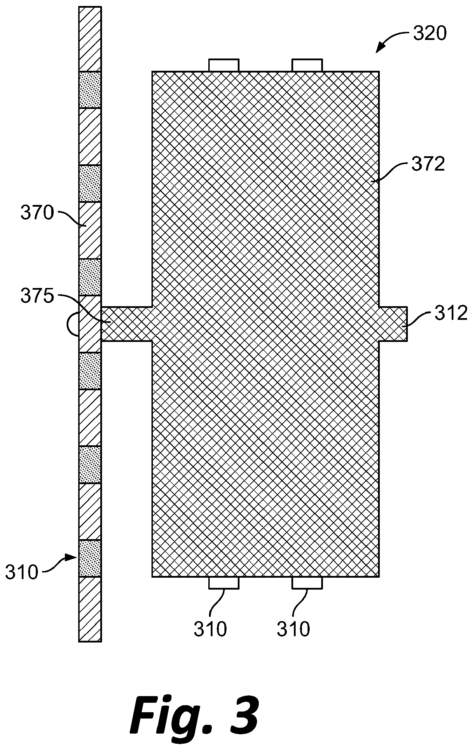

Fig. 3 is a schematic side view of an occluder 320 having electrodes 310 disposed around its surface. Occluder 320 is capable of ablating tissue in LAA 160 and plugging the LAA to reduce the risk of stroke due to atrial fibrillation. Occluder 320 includes a proximal disc 370 and a distal bulb 372 both made of a mesh including a plurality of strands, wherein at least one strand may be a metal strand. The strands may be braided, interwoven, or otherwise combined to define a generally tubular mesh. While the illustrated embodiment shows occluder 320 in an expanded state, the occluder preferably is formed of a shape-memory material that enables it to be compressed within a delivery device and to return to its expanded shape when released from the delivery device. A connective element 375 operably connects the proximal end of distal bulb 372 to proximal disc 370. The connection of connective element 375 to distal bulb 372 and proximal disc 370 may be configured such that the distal bulb and the proximal disc are articulable, rotatable, or otherwise movable with respect to the connective element and each other. Proximal disc 370 and distal bulb 372 are shaped and sized to fit snugly within LAA 160 when fully expanded. That is, proximal disc 370 and distal bulb 372 preferably have a diameter in the fully expanded condition that is larger than the diameter of LAA 160 so that occluder 320 will be frictionally held in the LAA. Occluder 320 may be anchored within LAA 160 by retention members (not shown) disposed around the surface of proximal disc 370 and/or distal bulb 372 that latch onto the tissue of the LAA.

Electrodes 310 may be spaced evenly around the perimeters of proximal disc 370 and distal bulb 372. Electrodes 310 conduct ablation energy to the surrounding tissue of LAA 160 to ablate a sufficient amount of the tissue to electrically isolate the LAA from the left atrium 122. Electrodes 310 are electrically connected to an energy source through a delivery device as will be described below. The energy source may supply radiofrequency, microwave, ultrasonic, electrical conduction or other types of energy, or combinations thereof, to electrodes 310. Electrodes 310 may be separated from anchor features, such as retention members, to help ensure that the tissue with which the anchor features engage is not directly affected by ablation (which could cause loss of tissue integrity and a weakened anchorage). For embodiments wherein electrodes 310 are rectangular or oblong, the orientations of adjacent electrodes may be alternated (radial versus longitudinal) around the perimeter of occluder 320. It is also contemplated that in some embodiments, electrodes 310 will only be placed around proximal disc 370. Each electrode 310 may be individually monitored and controlled so that different voltages may be applied to individual electrodes based upon monitored feedback. Some electrodes 310 may be larger than others to contact a larger surface area of LAA 160 in certain locations. Occluder 320 may also include at least one distally-located testing electrode 312. Testing electrodes 312 contact LAA tissue and connect to a device for measuring feedback through a wire extending through the delivery device. Testing electrodes 312 are capable of detecting an electrical impulse within LAA tissue to determine whether the application of ablation energy to LAA 160 has electrically isolated the LAA from left atrium 122. The loss of an electrical signal where testing electrodes 312 contact the LAA tissue indicates electrical isolation of LAA 160 from left atrium 122. After ablation is complete, the delivery device may be disconnected from occluder 320, leaving the occluder in place within LAA 160.

It is also contemplated that occluder 320 may be provided in different expanded sizes to form a friction fit within different shapes and sizes of LAAs and other cavities. It will be appreciated that even though the disclosure herein references an occlusion device comprising proximal disc 370 and distal bulb 372, such a configuration is for exemplary purposes only, and an occlusion device according to the other embodiments of the disclosure may comprise three or more separate and discrete portions configured to be cooperable according to the various principles disclosed herein. The occlusion device may also comprise only a single laterally-expandable body having any shape fit to fill the LAA cavity, such as a mushroom shape. Rather than the retention members described above, some embodiments of the occluder may be anchored by independent or integrated repositionable anchors, by barbs, and/or by distal anchoring elements.

Exemplary shape-memory materials for forming occluder 320 may include nitinol and shape-memory polymers (e.g., polyurethanes; polyurethanes with ionic or mesogenic components made by prepolymer method; and other block copolymers, such as block copolymers of polyethylene terephthalate (PET), polyethyleneoxide (PEO), block copolymers containing polystyrene and poly(1, 4-butadiene), and ABA triblock copolymers made from poly(2-methyl-2-oxazoline) and polytetrahydrofuran). Other materials for forming proximal disc 370 and distal bulb 372 include stainless steel, titanium, Elgiloy^{®}, Hastelloy^{®}, CoCrNi alloys (e.g., trade name Phynox), MP35N, CoCrMo alloys, or a mixture of metal and polymer fibers. The outer surfaces of proximal disc 370 and distal bulb 372 may be at least partially coated with a substance that electrically insulates these bodies. The coating may be a layer of polytetrafluoroethylene (PTFE).

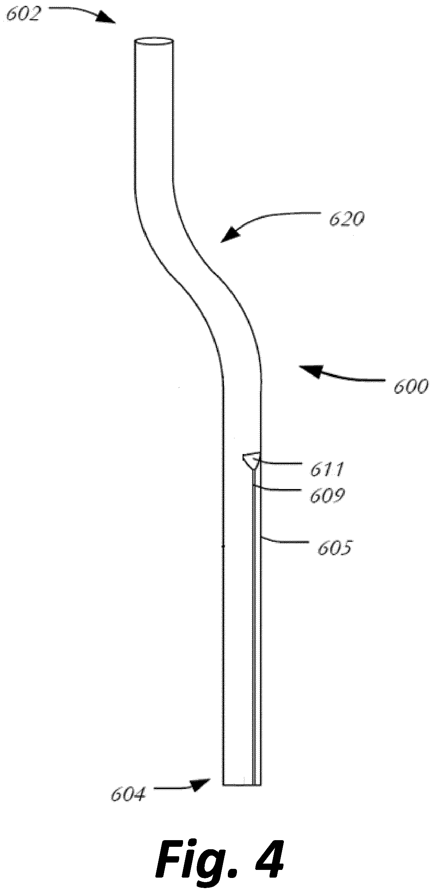

Fig. 4 illustrates a delivery device 600 for delivering occluder 320 to the vicinity of LAA 160 for deployment into the LAA. Delivery device 600 includes a steerable catheter 605 that extends between a distal end 602 and a proximal end 604, wherein the proximal end remains outside the patient. Catheter 605 has a lumen therethrough that is sized to receive an inner rod 609, occluder 320, wires (not shown) connecting an energy source (not shown) at proximal end 604 to electrodes 310 dispersed along the occluder, and a piercing guidewire (not shown) to pierce through tissue, such as, for example, the interatrial septum 152 for transseptal delivery as previously described. Catheter 605 may include a flexible curved portion 620 adjacent distal end 602 to aid in delivery. Inner rod 609 is disposed within catheter 605 and is translatable relative thereto. Inner rod 609 terminates in a plunger 611, which may comprise an enlarged head as shown in Fig. 4 for purposes of applying pressure to the proximal end of occluder 320 to translate it distally out from catheter 605. Alternatively, inner rod 609 may include another structure for translating occluder 320 out from catheter 605, such as a magnet, fastener, or a blunt tip. Catheter 605 may be formed of any known material for building catheters, including biocompatible metals such as stainless steel.

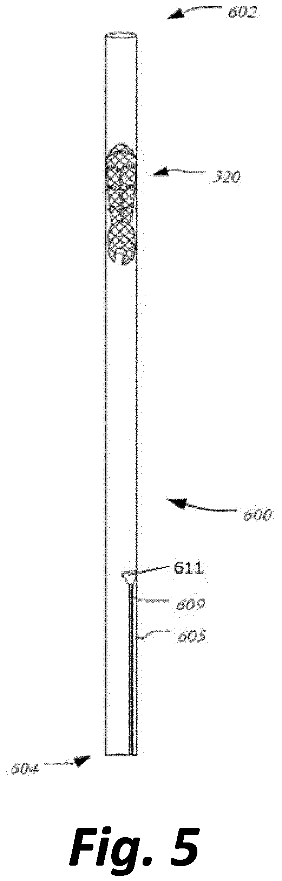

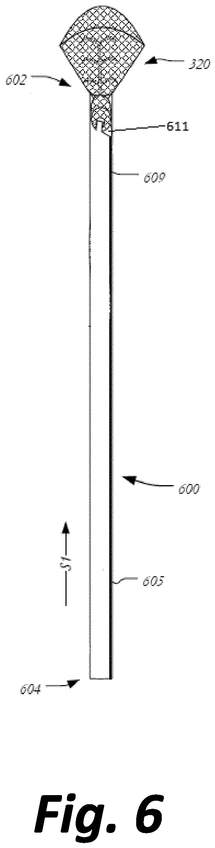

Fig. 5 illustrates delivery device 600 with occluder 320 releasably disposed within catheter 605. As an initial step as described above, the steerable catheter 605 may be directed through the venous system (e.g., the jugular vein or the femoral vein) of the patient to reach the right atrium 112. The piercing guide wire may be used to pierce interatrial septum 152 at position P2 to create an entry point for advancing delivery device 600 into left atrium 122 and to the site of LAA 160. Alternatively, a small incision may be made between the ribs and delivery device 600 may be advanced through the apex of left ventricle 124 at position P1 in heart wall 150 and through mitral valve 130 to reach left atrium 122 and LAA 160. Prior to deployment from catheter 605, occluder 320 is contained within the lumen of catheter 605 in a low-profile configuration. With distal end 602 of delivery device 600 disposed in LAA 160, occluder 320 may be urged forward in the direction of arrow S1 through catheter 605 using plunger 611, as shown in Fig. 6. For the sake of clarity, Fig. 6 does not show the patient anatomy. As plunger 611 is pushed distally relative to catheter 605, occluder 320 begins to deploy out from distal end 602. In the partially deployed state shown, only a portion of occluder 320 is outside of catheter 605. Moreover, the portion of occluder 320 that is disposed outside of catheter 605 has begun to expand to its relaxed expanded condition. With continued deployment, the entirety of occluder 320 will be exposed outside of catheter 605 and will expand within LAA 160. When occluder 320 expands, retention members (not shown) latch onto the tissue of LAA 160.

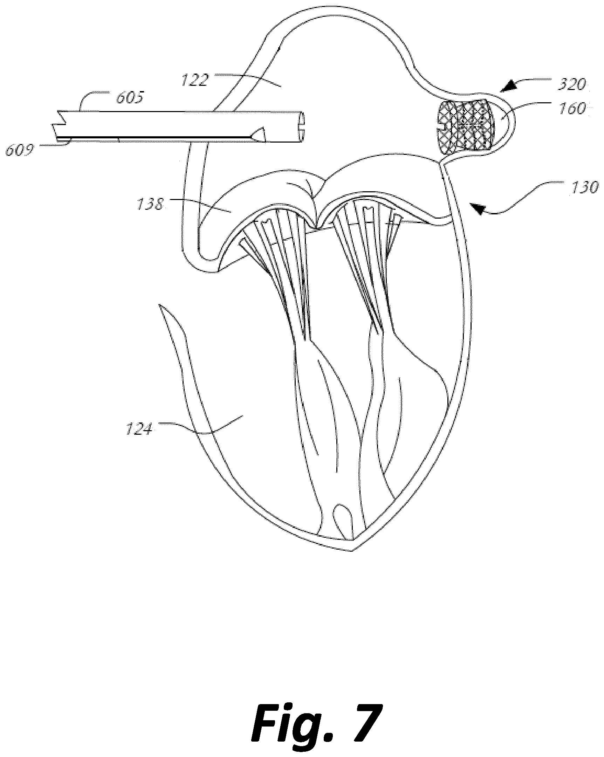

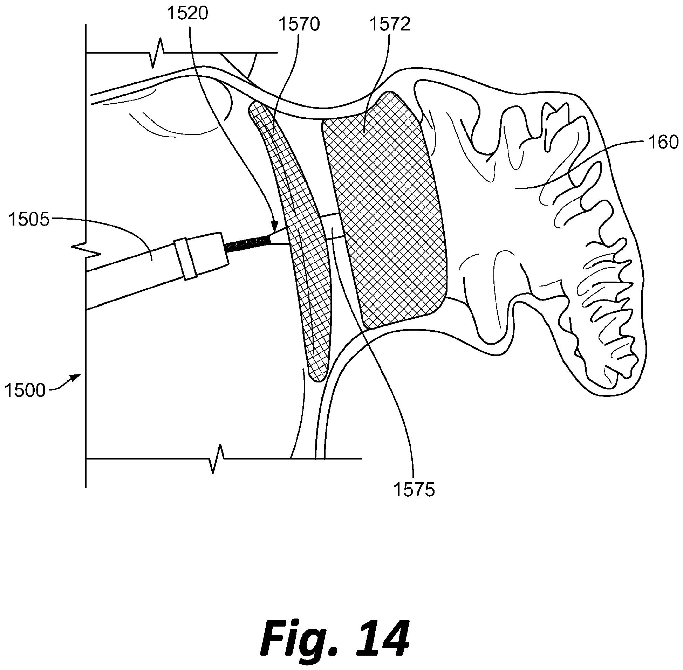

Fig. 7 illustrates a fully deployed occluder 320 secured within LAA 160. Following deployment, electrodes 310 and testing electrodes 312 are still connected by wires (not shown) to an energy source and a device capable of measuring feedback, respectively. As occluder 320 expands and electrodes 310 contact the tissue of LAA 160, ablation energy may be delivered to the tissue. After a period of time, ablation may be interrupted and testing electrodes 312 may be utilized to determine whether LAA 160 has been electrically isolated from left atrium 122. If not, ablation may be resumed until electrical isolation of LAA 160 has been achieved according to feedback from testing electrodes 312, at which point ablation may be terminated. Once ablation has been completed, the wires may be disconnected from electrodes 310, 312 by the retraction of delivery device 600. For example, wires may extend through a lumen in the catheter which may be coupled to the proximal end of the occluder by means of, *e.g.,* threaded surfaces as illustrated in Fig. 14. The wires may be coupled to the electrodes while the occluder is being delivered, and the wires may be detached from the electrodes after the occluder is delivered and the delivery device is unscrewed from the occluder and retracted from the body. The delivery device may be removed from the patient, leaving only occluder 320 in place to prevent clots from dislodging from LAA 160 and causing a stroke.

In some embodiments, the fully expanded diameter of occluder 320 is larger than the diameter of LAA 160 such that the occluder substantially fills (and may slightly stretch) the LAA. On the other hand, ablation energy may also be delivered to enhance the contact between occluder 320 and the surrounding tissue, and/or to enhance the anchoring (migration resistance) between the occluder and the surrounding tissue. In some implementations, gaps between occluder 320 and the surrounding tissue may exist when the occluder is deployed in LAA 160. That may be the result when, for example, the shape of LAA 160 is irregular to the extent that occluder 320 is unable to fully conform to the irregular tissue topography that surrounds it. When ablation energy is applied from the electrodes 310 of occluder 320 in the area of such gaps, scar tissue may form so as to fill the gaps. In effect, a positive remodeling of LAA 160 tissue may occur as a result of the ablation energy delivered by occluder 320, enhancing the contact between the occluder and the surrounding tissue.

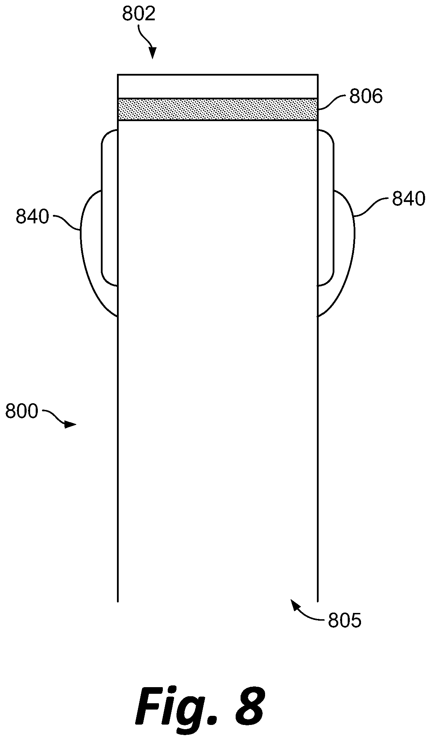

Fig. 8 illustrates the distal end of a delivery device 800 similar to delivery device 600 described above. Delivery device 800 includes a steerable catheter 805 with a balloon 840 coupled to the catheter adjacent distal end 802. Balloon 840 may be noncompliant, but preferably is compliant. Balloon 840 is shown in a deflated state in Fig. 8. While in a deflated state, balloon 840 will remain substantially flush against the outer surface of catheter 805 to avoid impeding the advancement of the catheter as it is delivered to the vicinity of LAA 160 through one of the above-described methods, e.g., transapically or transseptally. While balloon 840 is in a deflated state, catheter 805 will typically have an approximately 14 French outer diameter. As discussed further below, balloon 840 may be inflated at the discretion of a user as delivery device 800 reaches the vicinity of LAA 160. Balloon 840 may be inflated to an appropriate diameter until the outer surface of the balloon contacts the ostium of LAA 160. Alternatively, if balloon 840 is non-compliant, the balloon will inflate to a preselected diameter. Catheter 805 may further include a radiopaque marker band 806 extending around the circumference of the distal end 802 of catheter 805 to assist the user in tracking delivery device 800 as it is maneuvered through heart 100 to LAA 160. Rather than a single toroidal balloon 840 encircling catheter 805, delivery device 800 may include a plurality of balloons (*e.g.,* at least two balloons) spaced around the circumference of the catheter, and may expand and fill a substantially similar amount of space as a single balloon. The balloons may be compliant or noncompliant, or a mixture of compliant and noncompliant.

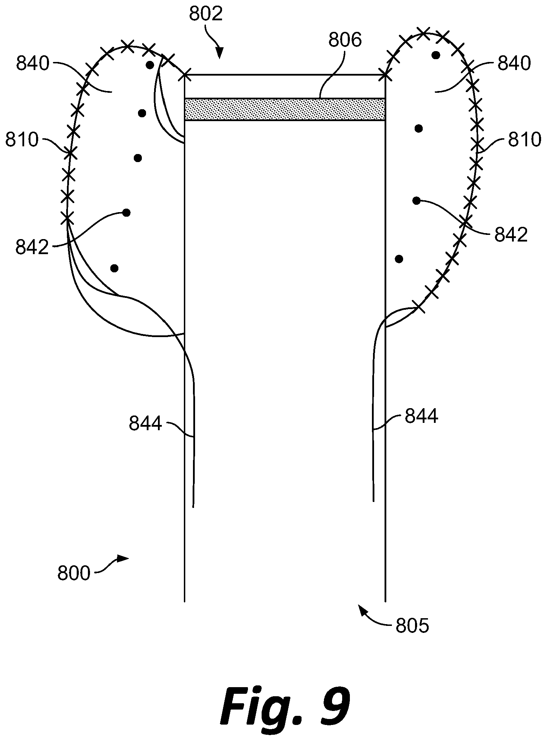

Fig. 9 is a schematic side view of the distal end of catheter 805 with balloon 840 in an inflated state. Balloon 840 may be inflated from a source of a gas, such as helium, outside the patient's body. The gas may be injected through a hub or y-shaped connector and into a tube (not shown) that extends through catheter 805, forming a secondary lumen within the catheter, and emerging from the catheter to reach the proximal end of the balloon. Catheter 805 may further include an additional tube (not shown) forming a third lumen that emerges from the catheter to introduce a fluid, such as saline, through micro-holes 842 in balloon 840 to irrigate the LAA and promote ablation. In the illustrated embodiment, electrodes 810 are coupled to the outer surface of balloon 840. Electrodes 810 may attach to balloon 840 by any appropriate means, such as by an adhesive. Electrodes 810 may be spaced evenly around the outer surface of balloon 840, or may be positioned in any manner that promotes contact between the electrodes and LAA 160 when the balloon is in an inflated state. Similar to embodiments described above, wires 844 may extend from electrodes 810 into the lumen of catheter 805 through a bore in the side surface of the catheter and through the catheter to an energy source located outside of the patient's body. In other embodiments, electrodes 810 may be coupled to a mesh netting that surrounds balloon 840, which will be discussed below in further detail. Also in other embodiments, the substance used to inflate balloon 840 may be carried in a container within the lumen of catheter 805 or attached to catheter 805 and injected into balloon 840 upon arrival at LAA 160.

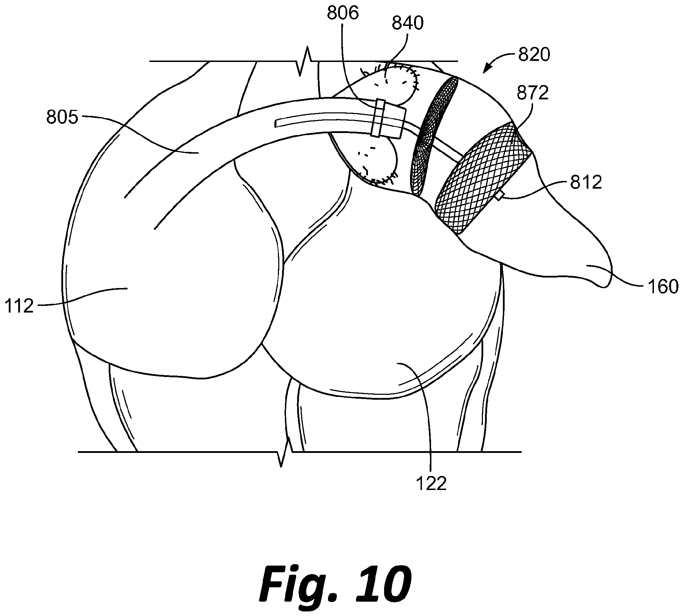

Electrodes 810 may be used to ablate tissue of LAA 160 in a manner similar to that described above. In the illustrated embodiment, occluder 820 (shown in Fig. 10) is disposed within catheter 805 of delivery device 800 prior to deployment. Catheter 805 may be steered by a user toward the opening of LAA 160, and balloon 840 may be inflated until electrodes 810 positioned around the surface of the balloon contact the tissue of the LAA. Ablation energy may then be delivered to the LAA tissue via electrodes 810, ablating the tissue in contact with the electrodes. Occluder 820 may then be deployed from catheter 805 of delivery system 800 into LAA 160. Balloon 840 may remain inflated while deploying occluder 820 from catheter 805 to apply pressure to the walls of LAA 160 to stabilize delivery device 800 while the occluder is being deployed, and may subsequently be deflated by draining the inflation substance back through the same tube used to inflate the balloon. After deployment of occluder 820 and deflation of balloon 840, catheter 805 may be withdrawn and removed from the patient.

Alternatively, balloon 840 may be deflated by draining the inflation substance prior to deploying occluder 820 from catheter 805. It is also contemplated that a testing electrode located on balloon 840 may be utilized in substantially the same manner as testing electrodes 312 described above to determine whether LAA 160 has been electrically isolated from left atrium 122 prior to deploying occluder 820 from catheter 805.

In addition to delivering ablation energy to electrically isolate LAA 160 from left atrium 122, ablation energy may also be delivered to enhance the contact between occluder 820 and the surrounding tissue, and/or to enhance the anchoring (migration resistance) between occluder 820 and the surrounding tissue in substantially the same manner as described above.

In some implementations, balloon 840 may be used to measure LAA 160 to determine the size of the occluder to be implanted. The expansion size of balloon 840 may be measured based on the volume of the substance, e.g., saline, injected into the balloon by the user. In some embodiments, balloon 840 may include pressure sensing features that detect a force applied to the balloon while it is expanded within LAA 160. Measuring the size of LAA 160 may be critical for delivering an appropriately sized occluder to ensure a proper fit within LAA 160. A pressure sensor (not shown) may be positioned on one or both lateral sides of balloon 840, and may be electrically connected via a wire to an electronic element having the capability of interpreting and storing the signals generated by the sensor. Alternatively, these signals may be transmitted wirelessly.

Fig. 10 is an illustration of delivery device 800 extending through heart 100 and approaching LAA 160 to ablate the LAA and deploy occluder 820 therein. Delivery device 800 is shown extending from right atrium 112 through the transseptal wall into left atrium 122 and resting at the entrance of LAA 160. It should be recognized that although occluder 820 is shown to have been deployed and balloon 840 inflated, catheter 805 was maneuvered through heart 100 in an initial state with the occluder compressed and disposed within the catheter and the balloon deflated. When the distal end 802 of delivery device 800 reaches the entrance to LAA 160, the user may inflate balloon 840, as described above, preferably until it contacts a substantial amount of LAA tissue. Electrodes 810 coupled to the outer surface of balloon 840 may ablate the tissue of LAA 160, the balloon may be deflated, and occluder 820 may subsequently be deployed from the catheter.

Alternatively, occluder 820 may be deployed into LAA 160 while balloon 840 is inflated. There may be sufficient space to deploy occluder 820 and inflate balloon 840 to contact LAA tissue as illustrated in Fig. 10, thus enabling the occluder to be deployed before or simultaneously with the ablation of LAA 160. It is also contemplated that a testing electrode 812 may be included at the distal-most end of distal bulb 872 or coupled to the surface of balloon 840 to engage the LAA tissue in the same testing procedure as described above.

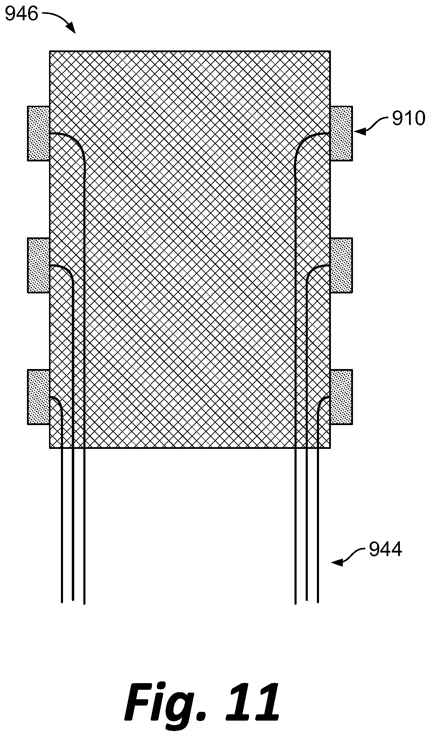

Fig. 11 illustrates a flattened woven mesh 946 positionable over the balloon 940 of delivery device 900 to secure electrodes 910 to the balloon. When completely stretched out in three dimensions, woven mesh 946 may assume a cylindrical shape, but any shape suitable for the woven mesh to encircle balloon 940 is acceptable. Woven mesh 946 may comprise an elastic fabric that contours to the shape of balloon 940 and expands with the balloon as the balloon inflates. Electrodes 910 may be fastened to woven mesh 946 by any suitable means. For example, electrodes 910 may be sutured to woven mesh 946.

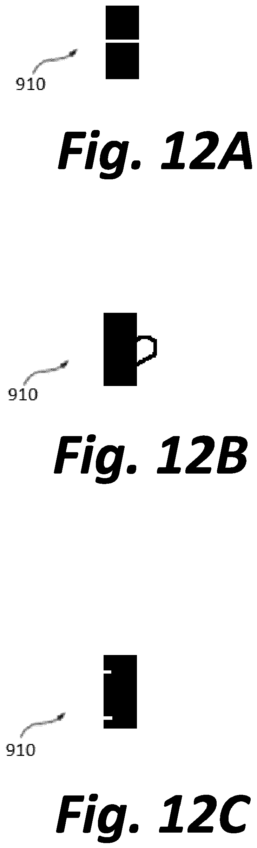

Figs. 12A-12C illustrate embodiments of electrodes 910 that facilitate suturing of the electrodes to woven mesh 946. Fig. 12A shows a cross-sectional view of one embodiment of electrode 910 having a bore extending through the middle thereof from one face to another, enabling the electrode to be sutured to woven mesh 946 through the bore. Fig. 12B shows a cross-sectional view of another embodiment of electrode 910 having a ring formed on or coupled to one face thereof such that the electrode may be sutured to woven mesh 946 through the ring. Fig. 12C shows a cross-sectional view of still another embodiment of electrode 910 having guide bores etched into one side thereof, whereby the electrode may be attached to woven mesh 946 by applying a suture through the guide bores and around the electrode to lace through the woven mesh along the face opposite the guide bores. Alternatively, electrodes 910 may be affixed to woven mesh 946 via an adhesive or printed onto the woven mesh as part of an electrical circuit.

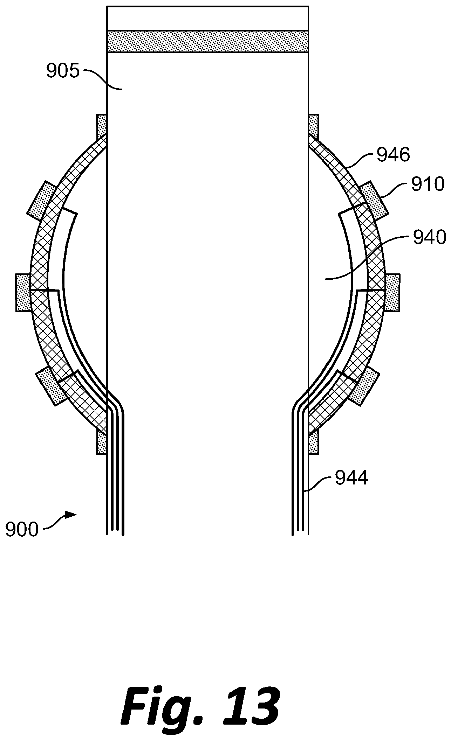

In the embodiment illustrated in Figs. 11 and 13, wires 944 extend proximally from electrodes 910, through woven mesh 946 and between the woven mesh and the surface of balloon 940 to reduce direct contact between the wires and the internal wall of LAA 160. The wires then pass through a bore (not shown) in the side surface of catheter 905 proximal to balloon 940 and extend proximally through the lumen of the catheter until they ultimately attach to an energy source (not shown). Wires 944 may also be insulated to reduce direct contact between the wires and LAA 160. Wires 944 may be coupled to electrodes 910 by any suitable means, e.g., by soldering, adhesives, laser welding and the like.

Fig. 13 illustrates a delivery device 900 that operates in substantially the same manner as delivery device 800 described above. As shown, the balloon 940 of delivery device 900 is in an inflated or expanded condition and includes woven mesh 946 contoured around the surface of the balloon with electrodes 910 sutured to the woven mesh. The procedure for ablation and occlusion may proceed in the same manner as described above in connection with delivery device 800.

Fig. 14 illustrates a delivery device 1500 configured to ablate LAA 160 via irreversible electroporation (IRE). Delivery device 1500 is configured to deliver an occluder 1520 in substantially the same manner as the delivery devices described above, e.g., using a steerable catheter 1505 to reach left atrium 122 either transseptally or transapically, then deploying the occluder from the distal end of the catheter with the use of a plunger. Although not shown, delivery device 1500 further includes a wire that extends distally from an energy source at the proximal end of the delivery device and through the lumen of catheter 1505 to reach occluder 1520. The wire then travels through a lumen in proximal disc 1570 and connector 1575 which define a pathway for the wire to reach distal bulb 1572. Proximal disc 1570 and distal bulb 1572 are comprised of a conductive metal mesh similar to embodiments described above. The present embodiment lacks an electrically insulating coating, enabling occluder 1520 to conduct energy directly to the surrounding tissue of LAA 160. After occluder 1520 has been delivered to and laterally expanded within LAA 160, a user may activate the energy source to deliver short pulses of high voltage energy, e.g., about 3000 volts, through the wire to distal bulb 1572 to ablate the LAA with IRE.

This arrangement eliminates the need to couple electrodes to the outer surface of occluder 1520 or to a balloon. This arrangement further eliminates the need for direct contact with the tissue of LAA 160 because the application of short bursts of high voltage energy enables ablating energy to jump across micro-gaps between occluder 1520 and the tissue of LAA 160, ablating all tissue immediately surrounding the occluder. The wire conducting the energy to occluder 1520 may be substantially insulated along the portion that extends from the energy source to distal bulb 1572, such that the wire contacts and delivers ablation energy only to the distal bulb. After ablation has been completed, the wire may be detached from occluder 1520 by the force applied from retraction of delivery device 1500, enabling the wire to be retracted while leaving the occluder in place. Typically, the ablation process via IRE may be completed in a matter of hundreds of microseconds to about a millisecond after occluder 1520 has been deployed in LAA 160. During this timeframe, multiple voltage pulses may be applied to ensure that sufficient tissue ablation has occurred. It is also contemplated to include a feedback module, such as a testing electrode on occluder 1520, to measure an electrical signal from LAA 160, similar to the manner described above.

In one variant of the foregoing embodiment, the wire may extend through the lumen of catheter 1505 and around the outer surface of occluder 1520. In another variant, the wire may be substantially insulated between the energy source and proximal disc 1570, thus contacting both the proximal disc and distal bulb 1572 to deliver ablation energy through both the proximal disc and distal bulb.

To summarize the foregoing, the present disclosure describes a heart treatment device, including a catheter extending from a proximal end to a distal end; an occluder releasably disposed within the catheter and adapted to be deployed within a left atrial appendage of a patient; a balloon coupled to the catheter; and an ablation electrode disposed on the balloon and configured to deliver ablation energy to tissue of the left atrial appendage; and/or
the heart treatment device may further include a plunger slidably disposed within the catheter, wherein sliding movement of the plunger toward the distal end of the catheter deploys the occluder from the catheter; and/or
the ablation electrode may be coupled to a surface of the balloon by an adhesive; and/or
the heart treatment device may further include a mesh overlying the balloon; and/or
the ablation electrode may be coupled to the mesh by a suture; and/or
the heart treatment device may further include a wire extending from the ablation electrode, between the mesh and the balloon, through a bore in the wall of the catheter, and through a lumen of the catheter to an energy source; and/or
the wire may be soldered or laser welded to the ablation electrode; and/or
the heart treatment device may further include a test electrode coupled to the occluder and configured to measure electrical signals in the tissue of the left atrial appendage; and/or
the distal end of the catheter may include a radiopaque marker; and/or
ablation energy may be delivered via an irreversible electroporation pathway; and/or
the balloon includes at least two balloons spaced around a circumference of the catheter; and/or
the catheter includes a tube forming a lumen that emerges from the catheter to introduce a fluid into the balloon through micro-holes in the balloon; and/or
the balloon includes a pressure sensor that detects a force applied to the balloon when the balloon is expanded to measure the size of the left atrial appendage; and/or
the electrode has an oblong shape extending a length in a first dimension and a width in a second dimension, the length greater than the width and the electrode oriented such that the length of the electrode extends along a longitudinal direction of the balloon; and/or
the electrode has an oblong shape extending a length in a first dimension and a width in a second dimension, the length greater than the width and the electrode oriented such that the length of the electrode extends along a radial direction of the balloon.

The present disclosure further describes a heart treatment device, including a catheter extending from a proximal end to a distal end; an occluder disposed within the catheter and adapted to be deployed from the distal end of the catheter within a left atrial appendage of a patient; an ablation electrode disposed on the occluder and configured to deliver ablation energy to tissue of the left atrial appendage; and a conductive wire connecting the electrode to an energy source; and/or
the heart treatment device may further comprise a test electrode coupled to the occluder and configured to measure electrical signals in the tissue of the left atrial appendage; and/or
the occluder includes a disc and a bulb connected to the disc by a connective element, the disc and bulb articulable relative to each other and the connective element, and the electrode positioned on the bulb; and/or
the electrode has an oblong shape extending a length in a first dimension and a width in a second dimension, the length greater than the width and the electrode oriented such that the length of the electrode extends along a longitudinal direction of the occluder; and/or
the electrode has an oblong shape extending a length in a first dimension and a width in a second dimension, the length greater than the width and the electrode oriented such that the length of the electrode extends along a radial direction of the occluder.

The present disclosure further describes a method for ablating tissue of the left atrial appendage of a patient and delivering a heart treatment device to the left atrial appendage. The method includes navigating a catheter through a patient's body to approach the left atrial appendage, the catheter having a distal end, a balloon coupled to the distal end, an ablation electrode disposed on the balloon, and an occluder disposed within a lumen of the catheter; inflating the balloon until the electrode contacts tissue of the left atrial appendage; ablating the tissue of the left atrial appendage using ablation energy delivered to the ablation electrode; and deploying the occluder from the lumen of the catheter into the left atrial appendage; and/or
the navigating step may include advancing the catheter through a septum between the left atrium and the right atrium of the patient; and/or
the navigating step may include advancing the catheter through the apex of the patient's heart; and/or
the inflating step may include injecting a volume of saline into the balloon; and/or
the method may further include measuring electrical signals in the left atrial appendage using a test electrode; and/or
the step of deploying the occluder may occur prior to the ablating step; and/or
the step of deploying the occluder may occur after the ablating step.

The present disclosure also describes a method for ablating tissue of the left atrial appendage of a patient and delivering a heart treatment device to the left atrial appendage. The method includes navigating a catheter through a patient's body to approach the left atrial appendage; deploying an occluder disposed within a lumen of the catheter into the left atrial appendage; and delivering ablation energy to the left atrial appendage through a wire connecting an energy source to an electrode in the left atrial appendage.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

It will be appreciated that the various dependent claims and the features set forth therein can be combined in different ways than presented in the initial claims. It will also be appreciated that any of the features described in connection with individual embodiments may be shared with others of the described embodiments.

## Claims

1. A heart treatment device (800), comprising:
a catheter (805) extending from a proximal end to a distal end (802);
an occluder (820) releasably disposed within the catheter (805) and adapted to be deployed within a left atrial appendage (160) of a patient;
a balloon (840) coupled to the catheter (805); and
an ablation electrode (810) disposed on the balloon (840) and configured to deliver ablation energy to tissue of the left atrial appendage (160).

2. The heart treatment device (800) of claim 1, further comprising a plunger (611) slidably disposed within the catheter (805), wherein sliding movement of the plunger (611) toward the distal end (802) of the catheter (805) deploys the occluder (820) from the catheter (805).

3. The heart treatment device (800) of claim 1, wherein the ablation electrode (810) is coupled to a surface of the balloon (840) by an adhesive.

4. The heart treatment device (900) of claim 1, further comprising a mesh (946) overlying the balloon (940).

5. The heart treatment device (900) of claim 4, wherein the ablation electrode (910) is coupled to the mesh (946) by a suture.

6. The heart treatment device (900) of claim 5, further comprising a wire (944) extending from the ablation electrode (910), between the mesh (946) and the balloon (940), through a bore in the wall of the catheter (905), and through a lumen of the catheter (905) to an energy source.

7. The heart treatment device (900) of claim 6, wherein the wire (944) is soldered or laser welded to the ablation electrode (910).

8. The heart treatment device (800) of claim 6, further comprising a test electrode (812) coupled to the balloon (840) and configured to measure electrical signals in the tissue of the left atrial appendage (160).

9. The heart treatment device (800) of claim 1, wherein the distal end (802) of the catheter (805) includes a radiopaque marker (806).

10. The heart treatment device (1500) of claim 1, wherein ablation energy is delivered via an irreversible electroporation pathway.

11. The heart treatment device (800) of claim 1, wherein the balloon 840 includes at least two balloons spaced around a circumference of the catheter 805.

12. The heart treatment device (800) of claim 1, wherein the catheter (805) includes a tube forming a lumen that emerges from the catheter (805) to introduce a fluid into the balloon (840) through micro-holes (842) in the balloon (840).

13. The heart treatment device (800) of claim 1, wherein the balloon (840) includes a pressure sensor that detects a force applied to the balloon (840) when the balloon (840) is expanded to measure the size of the left atrial appendage (160).

14. The heart treatment device (800) of claim 1, wherein the electrode (810) has an oblong shape extending a length in a first dimension and a width in a second dimension, the length greater than the width and the electrode (810) oriented such that the length of the electrode (810) extends along a longitudinal direction of the balloon (840).

15. The heart treatment device (800) of claim 1, wherein the electrode (810) has an oblong shape extending a length in a first dimension and a width in a second dimension, the length greater than the width and the electrode (810) oriented such that the length of the electrode (810) extends along a radial direction of the balloon (840).
